# EUROPÄISCHE PATENTSCHRIFT

(11) **EP 0 607 594 B1**
(45) Veröffentlichungstag und Bekanntmachung des Hinweises auf die Patenterteilung: **10.03.1999**
(21) Anmeldenummer: 93120542.1
(22) Anmeldetag: 20.12.1993
(51) Int. Cl.: A61M 25/00, A61B 17/28

(54) **Medizinische Zange**
Medical pincer
Pince médicale

(30) Priorität: 21.12.1992 DE 4243294; 27.09.1993 DE 4332829
(43) Veröffentlichungstag der Anmeldung: 27.07.1994
(73) Patentinhaber: UROTECH MED. TECHNOLOGIE GmbH, D-83052 Bruckmühl (DE)
(72) Erfinder: Jentsch, Peter, D-82418 Murnau (DE)
(74) Vertreter: Strasse, Joachim, Dipl.-Ing.

(56) Entgegenhaltungen:
- DE-A- 4 006 673
- GB-A- 2 176 112
- US-A- 5 098 440
- US-A- 5 152 779

## Beschreibung

Die vorliegende Erfindung betrifft eine medizinische Zange, insbesondere zum Eindringen in Körperhöhlen mit einem im Querschnitt runden, flexiblen Zangenkörper, wobei der Zangenkörper an einem Ende eine Greifvorrichtung und am gegenüberliegenden Ende eine Betätigungsvorrichtung aufweist, wobei der Zangenkörper rohrenartig ausgebildet ist und innerhalb des Zangenkörpers ein Positionierungsdraht geführt ist.

Eine derartige medizinische Zange ist aus der DE 40 06 673 A1 bekannt. Sie ist als Kathetervorrichtung zur Biopsie ausgebildet und umfaßt eine Zangeneinrichtung und ein Endoskop. Der Katheter ist dabei als Zwei-Kanal-Katheter aufgebaut, wobei ein Kanal als Zangenkanal und der andere Kanal als Endoskopkanal dient. Die Zange wird mittels eines Betätigungsstranges betätigt.

Weitere medizinische Zangen zum Eindringen in Körperhöhlen sind bekannt. Sie werden insbesondere zur Entfernung oder Positionierung von Kathetern in Körperhöhlen, z. B. im Urogenitalbereich, verwendet. Gerade aber bei der Positionierung von Kathetern treten immer wieder Probleme auf, da die entsprechenden Einführungsöffnungen im Körper sehr kleine Durchmesser aufweisen und oftmals zusätzlich verengt sind. Um dennoch eine sichere Positionierung eines Katheters zu gewährleisten, wird ein zusätzlicher Positionierungsdraht verwendet. Der Positionierungsdraht weist dabei einen sehr viel geringeren Durchmesser als die bekannten medizinischen Zangen bzw. die zu positionierenden Katheter auf. Durch den geringen Durchmesser kann der Positionierungsdraht leichter in die entsprechenden Körperhöhlen eingeführt werden, nach Einlegen des Drahtes kann dann der Katheter mit Hilfe der bekannten medizinischen Zangen über den Positionierungsdraht in die Körperhöhle geführt werden.

Nachteilig an diesem Stand der Technik ist jedoch, daß hier, insbesondere beim Legen, Entfernen oder Positionieren von Kathetern, mindestens zwei Vorrichtungen, nämlich die bekannten medizinischen Zangen und ein separater zusätzlicher Positionierungsdraht benötigt werden. Dies bedeutet einen erhöhten Zeitaufwand, da hier mindestens zwei Einführungsvorgänge stattfinden müssen. Ein weiterer Nachteil ergibt sich aus der Notwendigkeit, daß die bekannten Positionierungsdrähte mit zusätzlichen Vorrichtungen eingeführt und gegebenenfalls arretiert werden müssen, wodurch die Handhabbarkeit des Drahtes erschwert wird.

Aufgabe der vorliegenden Erfindung ist es, eine medizinische Zange, insbesondere zum Eindringen in Körperhöhlen, bereitzustellen, die eine schnelle, einfache und sichere Positionierung von Kathetern in Körperhöhlen gewährleistet.

Zur Lösung dieser Aufgabe dienen die Merkmale des unabhängigen Anspruches.

Vorteilhafte Ausgestaltungen sind in den Unteransprüchen beschrieben.

Die erfindungsgemäße medizinische Zange gewährleistet durch einen röhrenartig ausgebildeten Zangenkörper, innerhalb dessen ein Positionierungsdraht geführt ist, eine schnelle und leichte Positionierung von Kathetern in Körperhöhlen. Durch die Führung des Positionierungsdrahtes innerhalb des Zangenkörpers kann vorteilhafterweise das Legen, Entfernen oder Positionieren von Kathetern in Körperhöhlen mit einem Einführungsvorgang, nämlich der Einführung der erfindungsgemäßen medizinischen Zange in den Körper vollzogen werden.

Die Greifvorrichtung besteht aus mindestens zwei Greifern, wobei die Greifer an einem ebenfalls in dem Zangenkörper geführten, flexiblen Innenrohr befestigt sind. Dadurch kann die erfindungsgemäße medizinische Zange in ihrem Aufbau klein gehalten werden.

In einer weiteren vorteilhaften Ausgestaltung besteht der Zangenkörper aus Metall, wobei er vorzugsweise aus einem schraubenlinienförmig gedrehten Metallstück besteht. Durch die schraubenlinienförmige Ausbildung des Zangenkörpers ist eine besonders leichte und sichere Positionierung der erfindungsgemäßen Zange in den Körperhöhlen möglich.

Vorteilhafterweise weist der Positionierungsdraht einen Durchmesser zwischen 0,025 bis 0,038 mm auf, wodurch gewährleistet ist, daß die üblicherweise verwendeten Katheter über den Draht geführt werden können.

In einer weiteren vorteilhaften Ausgestaltung weist die erfindungsgemäße medizinische Zange eine Betätigungsvorrichtung auf, die mindestens eine Zuführ- und Arretierungsvorrichtung für den Positionierungsdraht aufweist. Damit ist gewährleistet, daß der Positionierungsdraht auch nach Einführung der erfindungsgemäßen Zange in eine Körperhöhle eingeführt, ausgewechselt oder positioniert werden kann. Weiterhin ergibt sich vorteilhafterweise die Möglichkeit, daß der Positionierungsdraht bei geöffneter Arretierungsvorrichtung in der Körperhöhle positioniert bleiben kann, wenn der Zangenkörper aus der Körperhöhle entfernt wird.

Weitere Einzelheiten, Vorteile und Merkmale der Erfindung ergeben sich aus der nachfolgenden Beschreibung des in den Zeichnungen dargestellten, bevorzugten Ausführungsbeispiels.

Es zeigen:
- Figur 1:: Eine schematische Darstellung der erfindungsgemäßen medizinischen Zange und
- Figur 2:: eine schematische Darstellung der Greifvorrichtung der erfindungsgemäßen medizinischen Zange.

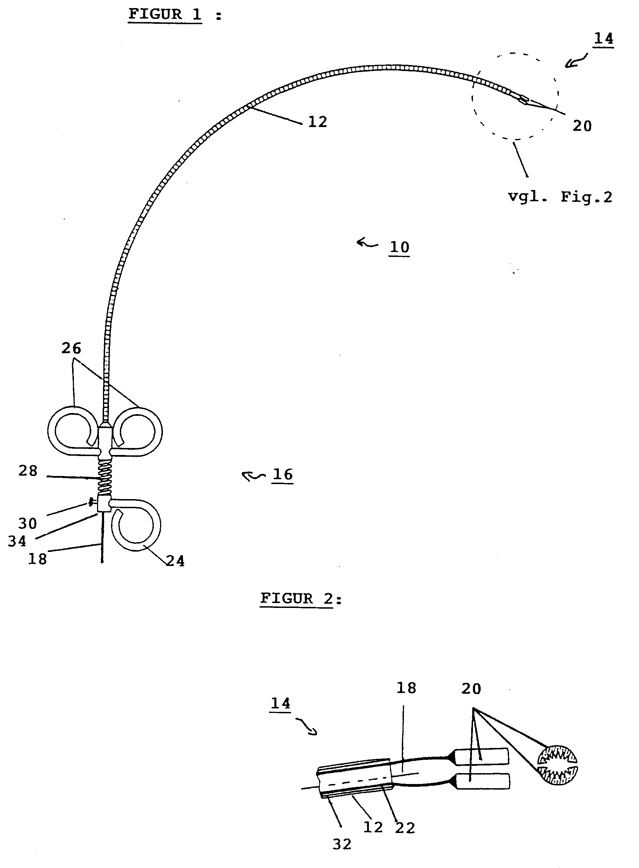

Die in Figur 1 schematisch dargestellte erfindungsgemäße medizinische Zange umfaßt einen Zangenkörper 12, wobei der Zangenkörper 12 an einem Ende eine Greifvorrichtung 14 und am gegenüberliegenden Ende eine Betätigungsvorrichtung 16 aufweist. Der Zangenkörper 12 ist flexibel ausgebildet und weist einen im Querschnitt runden, röhrenförmigen Aufbau auf. Der Zangenkörper 12 besteht in dem Ausführungsbeispiel aus einem schraubenlinienförmig gedrehten Metallstück. In einer weiteren Ausführungsform kann der Zangenkörper 12 auch aus Kunststoff bestehen, insbesondere Polyurethan oder Teflon.

In dem röhrenförmigen Zangenkörper 12 ist ein flexibles Innenrohr 22 geführt. An einem Ende des flexiblen Innenrohres 22 ist die Greifvorrichtung 14, welche mindestens aus zwei Greifern 20 besteht, befestigt. Das der Greifvorrichtung 14 gegenüberliegende Ende des Innenrohres 22 ist mit der Betätigungsvorrichtung 16 verbunden. Das flexible Innenrohr 22 besteht ebenfalls aus einem schraubenlinienförmig gedrehten Metallstück. Zur Vermeidung von Reibungsverlusten zwischen dem Zangenkörper 12 und dem Innenrohr 22 ist zwischen der inneren Oberfläche des Zangenkörpers 12 und der Außenfläche des Innenrohrs 22 eine Gleitverbindung 32 (vgl. Figur 2) ausgebildet. Die Gleitverbindung 32 besteht vorzugsweise aus einer flexiblen Kunststoffröhre.

Die Betätigungsvorrichtung 16 weist mindestens zwei Griffstücke 24, 26 auf, wobei zwischen den Griffstücken 24, 26 eine Rückstellvorrichtung 28 ausgebildet ist. Durch ein Gegeneinanderdrücken der Griffstücke 24 und 26 wird das mit der Betätigungsvorrichtung 16 verbundene Innenrohr 22 in Richtung der Greifvorrichtung 14 verschoben. Die an dem Innenrohr 22 befestigten Greifer 20 werden dadurch aus dem Zangenkörper 12 geführt und geöffnet. Durch die Rückstellvorrichtung 28 ist gewährleistet, daß anschließend die beiden Griffstücke 24 und 26 in ihre Ausgangslage zurückkehren, wobei die Greifer 20 wieder über das Innenrohr 22 in den Zangenkörper 12 gezogen werden und sich dabei schließen.

Die erfindungsgemäße medizinische Zange 10 umfaßt weiterhin einen Positionierungsdraht 18, der innerhalb des Zangenkörpers 12 geführt ist. Der Positionierungsdraht 18 weist einen Durchmesser zwischen 0,01 und 0,05 mm, vorzugsweise 0,025 bis 0,038 mm auf. Der Durchmesser ist dabei so gewählt, daß der Positionierungsdraht in einen nicht dargestellten, rohrförmigen Katheter eingeführt werden kann. Mit Hilfe des Positionierungsdrahts 18 kann ein Katheter in eine Körperhöhle gebracht, daraus entnommen oder darin positioniert werden.

Die Betätigungsvorrichtung 16 weist mindestens eine Zuführöffnung 34 und Arretierungsvorrichtung 30 für den Positionierungsdraht 18 auf. Durch die Zuführöffnung 34 ist gewährleistet, daß der Positionierungsdraht 18 jederzeit in das mit der Betätigungsvorrichtung 16 verbundene Innenrohr 22 eingeführt werden kann. Die Arretierungsvorrichtung 30 dient zur Fixierung des Drahtes 18 in einer gewünschten Lage.

Die Figur 2 zeigt die Greifvorrichtung 14 in größerem Detail. Die Greifvorrichtung 14 umfaßt dabei mindestens zwei Greifer 20. Die Greifer 20 sind jeweils schaufelartig ausgebildet und mit dem der Schaufelfläche gegenüberliegenden Seite mit dem Innenrohr 22 verbunden. In dem Ausführungsbeispiel wird das Öffnen der Greifvorrichtung 14 durch die Verschiebung des Innenrohrs 22 mittels der Betätigungsvorrichtung 16 in Richtung der Greifvorrichtung 14 getätigt. Die gegenläufige Bewegungsrichtung des Innenrohrs 22 bewirkt das Schließen der Greifer 20. Figur 2 zeigt auch einen Querschnitt durch die Greifer 20. Man erkennt, daß die Greifer 20 einen halbkreisförmigen Querschnitt aufweisen, wobei die Innenseite der Greifer 20 sägezahnartig ausgebildet ist.

## Patentansprüche

1. Medizinische Zange, insbesondere zum Eindringen in Körperhöhlen, mit einem im Querschnitt runden, flexiblen Zangenkörper (12), wobei der Zangenkörper (12) an einem Ende eine Greifvorrichtung (14) und am gegenüberliegenden Ende eine Betätigungsvorrichtung (16) aufweist, wobei der Zangenkörper (12) röhrenartig ausgebildet ist und innerhalb des Zangenkörpers (12) ein Positionierungsdraht (18) geführt ist,
**dadurch gekennzeichnet,**
daß die Greifvorrichtung (14) aus mindestens zwei Greifern (20) besteht, wobei die Greifer (20) an einem in dem Zangenkörper (12) geführten, flexiblen Innenrohr (22) befestigt sind.

2. Medizinische Zange nach Anspruch 1,
**dadurch gekennzeichnet,**
daß der Zangenkörper (12) aus einem schraubenlinienförmig gedrehten Metallstück besteht.

3. Medizinische Zange nach Anspruch 1,
**dadurch gekennzeichnet,**
daß der Zangenkörper (12) aus Kunststoff besteht.

4. Medizinische Zange nach Anspruch 3,
**dadurch gekennzeichnet,**
daß der Zangenkörper (12) aus Polyurethan oder Teflon besteht.

5. Medizinische Zange nach einem oder mehreren der vorhergehenden Ansprüche,
**dadurch gekennzeichnet,**
daß der Positionierungsdraht (18) einen Durchmesser zwischen 0,025 - 0,038 mm aufweist.

6. Medizinische Zange nach einem oder mehreren der vorhergehenden Ansprüche,
**dadurch gekennzeichnet,**
daß die Betätigungsvorrichtung (16) mindestens zwei Griffstücke (24, 26) aufweist, wobei zwischen den Griffstücken (24, 26) eine Rückstellvorrichtung (28) ausgebildet ist.

7. Medizinische Zange nach Anspruch 6,
**dadurch gekennzeichnet,**
daß die Rückstellvorrichtung (28) als Feder ausgebildet ist.

8. Medizinische Zange nach einem oder mehreren der vorhergehenden Ansprüche,
**dadurch gekennzeichnet,**
daß die Betätigungsvorrichtung (16) mindestens eine Zuführöffnung (34) und Arretierungsvorrichtung (30) für den Positionierungsdraht (18) aufweist.

9. Medizinische Zange nach einem oder mehreren der vorhergehenden Ansprüche,
**dadurch gekennzeichnet,**
daß zwischen der inneren Oberfläche des Zangenkörpers (12) und der Außenfläche des Innenrohrs (22) eine Gleitverbindung (32) ausgebildet ist.

10. Medizinische Zange nach Anspruch 9,
**dadurch gekennzeichnet,**
daß die Gleitverbindung (32) aus einer flexiblen Kunststoffröhre besteht.

## Claims

1. A medical pincer, in particular for penetrating body cavities, having a flexible pincer body (12) of circular cross-section, said pincer body (12), at one end thereof, including gripping means (14) and, at the opposite end thereof, actuation means (16), said pincer body (12) furthermore being of tube-like design, with a positioning wire (18) being guided within said pincer body (12)
**characterized in that**
said gripping means (14) includes at least two grippers (20), said grippers (20) being mounted on a flexible internal tube (22) guided within said pincer body (12).

2. The medical pincer of claim 1
**characterized in that**
said pincer body (12) comprises a helically turned metal piece.

3. The medical pincer of claim 1
**characterized in that**
the pincer body (12) is made of plastic.

4. The medical pincer of claim 3
**characterized in that**
the pincer body (12) is made of polyurethane or teflon.

5. The medical pincer of one or plural of the preceding claims
**characterized in that**
the positioning wire (18) is between 0.025 mm - 0.038 mm in diameter.

6. The medical pincer of one or plural of the preceding claims
**characterized in that**
the actuation means (16) includes at least two grip ends (24, 26), with a reset device (28) being provided between said grip ends (24, 26).

7. The medical pincer of claim 6
**characterized in that**
said reset device (28) is in the form of a spring.

8. The medical pincer of one or plural of the preceding claims
**characterized in that**
the actuation means (16) includes at least one introduction opening (34) and fixing device (30) for the positioning wire (18).

9. The medical pincer of one or plural of the preceding claims
**characterized in that**
a sliding connection (32) is provided between the inner surface of the pincer body (12) and the outer surface of the internal tube (22).

10. The medical pincer of claim 9
**characterized in that**
the sliding connection (32) comprises a flexible plastic tube.

## Revendications

1. Pince médicale, en particulier pour l'introduction dans des cavités du corps, avec un corps (12) de la pince flexible et de coupe transversale circulaire, le corps (12) de la pince présentant à une extrémité un mécanisme de prise à mors (14) et à l'extrémité opposée un mécanisme d'actionnement (16), le corps (12) de la pince étant en forme de tube et un fil de positionnement (18) étant inséré dans le corps (12) de la pince,
**caractérisée en ce que**
le mécanisme de prise à mors (14) est composé d'au moins deux mors (20), les mors (20) étant fixés à un tube (22) intérieur flexible et introduit dans le corps (12) de la pince.

2. Pince médicale suivant la revendication 1,
**caractérisée en ce que**
le corps (12) de la pince est composé d'une pièce de métal tournée en forme de pas de vis.

3. Pince médicale suivant la revendication 1,
**caractérisée en ce que**
le corps (12) de la pince est composé de matière plastique.

4. Pince médicale suivant la revendication 3,
**caractérisée en ce que**
que le corps (12) de la pince est composé de polyuréthane ou de téflon.

5. Pince médicale suivant l'une ou plusieurs des revendications précédentes,
**caractérisée en ce que**
le fil de positionnement (18) présente un diamètre situé entre 0,025 et 0,038 mm.

6. Pince médicale suivant l'une ou plusieurs des revendications précédentes
**caractérisée en ce que**
le mécanisme d'actionnement (16) présente au moins deux manettes (24, 26), un mécanisme de rappel (28) étant conçu entre les deux manettes (24, 26).

7. Pince médicale suivant la revendication 6,
**caractérisée en ce que**
le mécanisme de rappel (28) est conçu sous forme de ressort.

8. Pince médicale suivant l'une ou plusieurs des revendications précédentes,
**caractérisée en ce que**
le mécanisme d'actionnement (16) présente au moins une ouverture (34) d'introduction et au moins un mécanisme d'arrêt (30) du fil de positionnement (18).

9. Pince médicale suivant l'une ou plusieurs des revendications précédentes,
**caractérisée en ce que**
un joint de glissement (32) est disposé entre la paroi intérieure du corps (12) de la pince et la paroi extérieure du tube intérieur (22).

10. Pince médicale suivant la revendication 9,
**caractérisée en ce que**
le joint de glissement (32) est composé d'un tube flexible en matière plastique.
